Europäisches Patentamt

European Patent Office

Office européen des brevets

(19)

(11) Publication number: **0 001 694**
A1

(12)

# EUROPEAN PATENT APPLICATION

(21) Application number: 78300449.2

(22) Date of filing: 03.10.78

(51) Int. Cl.²: **C 07 C 29/28**
C 07 C 31/08

(30) Priority: 14.10.77 GB 42753/77

(43) Date of publication of application:
02.05.79 Bulletin 79/9

(84) Designated contracting states:
DE FR GB

(71) Applicant: BP Chemicals Limited
Britannic House Moor Lane
London, EC2Y 9BU(GB)

(72) Inventor: Cane, Charles
Hull Works Salt End Hedon
Hull North Humberside HU12 8DS(GB)

(74) Representative: Harry, John et al,
BP TRADING LIMITED Patents & Licensing Division
Chertsey Road
Sunbury-on-Thames Middlesex TW16 7LN(GB)

(54) Process for producing high purity anhydrous ethanol.

(57) The present invention is a process for producing substantially anhydrous ethanol of very high purity by azeotropically distilling aqueous ethanol with cyclohexane as an entrainer in a drying still, removing substantially all the water as an overhead fraction as a ternary azeotrope of water/cyclohexane/ethanol, and withdrawing anhydrous ethanol in the vapour phase either from the lower half of the drying still or as an overhead fraction from a further tailing still into which anhydrous liquid ethanol from the base of the drying still has been fed. This method prevents ethanol from being downgraded with respect to its permanganate time and Komarowski values owing to solvent extraction of impurities from flexible materials used for jointing purposes and pump seals. The final product is of very high purity and may be used in potable or pharmaceutical applications.

EP 0 001 694 A1

Croydon Printing Company Ltd.

1

## PROCESS FOR PRODUCING HIGH PURITY ANHYDROUS ETHANOL

The present invention relates to a process for purifying ethanol, especially for producing very high quality ethanol.

The drying of ethanol:water azeotrope, obtained either via olefin hydration or by fermentation processes, is conventionally accomplished by distillation using a hydrocarbon entrainer such as cyclohexane or benzene/n-heptane mixture, whereby a ternary azeotrope comprising water/hydrocarbon/ethanol is withdrawn overhead and substantially anhydrous ethanol final product withdrawn as a liquid from the base of the distillation column. Impurity levels in the ethanol are conventionally determined by permanganate time (PT; for oxidisable impurities such as acetaldehyde or diacetyl) and the Komarowski test (for higher alcohols especially tert-butanol). The ethanol final product obtained in the above manner is generally downgraded with respect to the above tests during drying by distillation, owing to solvent extraction of impurities from flexible materials used for jointing purposes and pump seals. Where the final product is to be used in potable or pharmaceutical applications, very high purity is required.

The effect on the PT and Komarowski tests of immersing various following materials (which are used in making seals in full scale plants) in (a) ethanol:water azeotrope and (b) in a mixture of ethanol and cyclohexane for 6 hours at ambient temperature is given below.

1

| Test Material Used (c) | (a) ethanol:water azeotrope | | (b) ethanol/cyclohexane mixture | |
|---|---|---|---|---|
| | Komarowski Value (ppm) | Permanganate Time (min) | Komarowski Value (ppm) | Permanganate Time (min) |
| Neoprene* | 22 | 35 | 458 | 10 |
| Silicon | 29 | 35 | 130 | 10 |
| Nylon | 41 | 30 | 137 | 10 |
| PTFE | 18 | 35 | 20 | 15 |
| Polythene* | 52 | 31 | - | - |
| Tygon R3603* | 500 | 12 | - | - |
| Tygon R4000* | 500 | 13 | - | - |

\* Registered Trade Mark

(a)   The untreated ethanol:water azeotrope had a Komarowski value of 22 ppm of tert-butanol equivalent and a PT of 35 minutes.

(b)   This test mixture comprised ca 20% w/w dried ethanol final product and 80% w/w cyclohexane (ie, approximately azeotrope composition), and this had a Komarowski value of 20 ppm of tert-butanol equivalent and a PT of 15 minutes.

Tubular pieces of the test material were used which were ca 2 cm long and 0.5 cm in diameter.

The results show that the extraction of impurities from the above materials is increased in the presence of cyclohexane and that minimum contamination of the ethanol is incurred with PTFE.

It has now been found that the above impurities in ethanol may be significantly reduced by collecting the ethanol final product as a vapour, instead of, as conventionally, as a liquid from the drying still.

Accordingly, the present invention is a process for producing substantially anhydrous ethanol of very high purity comprising azeotropically distilling aqueous ethanol together with cyclohexane as an entrainer in a drying still containing at least 50 plates, removing substantially all the water as an overhead fraction comprising a ternary azeotrope of water/cyclohexane/ethanol, and withdrawing anhydrous ethanol in the vapour phase either from a point intermediate between the level of liquid in the base of the drying still and the 5th plate or as an overhead fraction from a further tailing still into which anhydrous liquid ethanol from the base of the drying still has been fed.

The drying still is required to obtain an adequate separation as an overhead fraction of the heterogeneous ternary azeotrope of ethanol, cyclohexane and water from the homogeneous binary azeotrope of ethanol and water fed to the drying still. The difference in boiling point between these two azeotropes is $6.5^{\circ}C$ at 1.01 bar. The drying still column preferably contains at least 50 plates, most preferably between 60 and 70 plates.

The aqueous ethanol is suitably fed into the upper half of the drying still column, preferably at a point between 35 and 45 plates from the kettle at the base of the column. The overhead ternary azeotrope product may be condensed in a decanter. The temperature of the decanter is suitably below the boiling point of the overhead ternary azeotrope product, preferably below $60^{\circ}C$. The aqueous phase which separates as a result may be removed by decantation and then fed to a recovery still to recover the organic content for recycle.

The recovery still preferably contains between 30 and 40 plates. The aqueous phase separated from the drying still overheads (usually amounting to more than 0.3:1 of drying still feed by weight) is suitably fed to the lower half of the recovery still, preferably at a point between 7 and 20 plates from the kettle at the base of the column. The organic content of this phase is distilled overhead in the recovery still using a reflux ratio preferably in the range 5-10:1. The overhead distillate product from the recovery still may be recycled to the drying still column at any point intermediate between the wet ethanol feed point and the top drying still either by itself or combined with the organic upper phase from the decanter. Water is removed from the base of the recovery still.

The dried ethanol final product is collected as a vapour from the drying still column preferably from the base of the column. It is possible to achieve this by using conventional traps for excluding liquid. When, on the other hand, liquid is removed from the base of the still for feeding into a recovery still, traps to exclude vapour known in the art may be used.

It is desirable to remove a small purge from the kettle contents of the drying still to prevent build-up of impurities.

All flexible connections, gaskets and pump seals used in the ethanol drying and recovery stills are preferably made of solvent-resistant materials, such as for example polytetrafluorethylene (PTFE), polyethylene, polypropylene or nylon.

In a further embodiment pure, anhydrous ethanol is obtained by withdrawing anhydrous liquid ethanol from the base of the drying column and feeding said anhydrous liquid ethanol to the lower half of a further rectification or tailing still. The tailing still preferably contains between 40 and 50 plates. Vapours of pure anhydrous ethanol are withdrawn overhead from this tailing still. Preferably, the tailing still is operated at a reflux ratio within the range 1 to 20:1.

The process of the present invention is further illustrated with reference to the following Examples.

Example 1

Ethanol:water azeotrope (13.64 kg, which gave a permangante time of 35 minutes and a Komarowski test result of 22 ppm of tert-butanol equivalent) and cyclohexane (1 kg) was fed over 31 hours onto the 40th plate of a 58-plate Oldershaw column (50 mm diameter) drying still. Ethanol:cyclohexane:water ternary azeotrope (61.6 kg) was taken overhead, condensed and the two phases which formed were separated in a decanter at 38°C. Organic phase (56.3 kg) from the decanter was recycled to the top of the drying still column. Dry ethanol final product (9.4 kg) collected from the base of the drying still, contained 0.05% w/w water and 1 ppm of cyclohexane. A flash tailing distillation of an aliquot of the dry ethanol liquid final product above gave a permanganate time of 26 minutes and a Komarowski value of 28 ppm of tert-butanol equivalent. The flexible connections used in both stills were made of neoprene.

The collection of ethanol final product as a vapour instead of as a liquid from the base of the drying still gave similar results.

The aqueous phase (5.3 kg) from the drying still decanter was fed onto the 10th plate of a 35-plate Oldershaw column (50 mm diameter) recovery still. Ethanol:cyclohexane:water azeotrope mixture (4.7 kg) was taken overhead with a reflux ratio of 7:1 and this was recycled to be mixed with the feed to the top of the drying still column together with decanter organic phase. Water (0.6 kg) was removed from the base of the recovery still.

Comparative Test 1

In a comparative test, not according to the invention an aliquot of the dry ethanol liquid final product collected from the base of the drying still in Example 1 above gave a Komarowski test result of 800 ppm of tert-butanol equivalent. This indicated a significant down grading of the ethanol final product from the drying process without the flash tailing distillation step.

Example 2

A tailing distillation of another aliquot of the dried ethanol final product from Example 1 above through a 5-plate Oldershaw column, using a reflux ratio of 2:1 gave a permanganate time of 25 minutes and a Komarowski value of 20 ppm of tert-butanol equivalent.

Example 3

A tailing distillation of a third aliquot of the dried ethanol product from Example 1 above through a 40-plate Oldershaw column using a reflux ratio of 10:1 gave a permanganate time of 28 minutes and a Komarowski value of 1 ppm of tert-butanol equivalent.

Comparative Test 2 (not according to the invention)

In this test aqueous ethanol was azeotropically distilled with a cyclohexane entrainer as a in Example 1 but the flash tailing distillation step of the ethanol liquid final product was omitted.

The dried ethanol final product recovered as a liquid base product gave a permanganate time of 10 minutes and a Komarowski test result of ca 800 ppm of tert-butanol equivalent which again indicated that a significant downgrading in ethanol quality had resulted from the above drying process without the flash tailing distillation.

Comparative Test 3 (not according to the invention)

In this case, the flexible connections used in both the drying and recovery stills were made of polytetrafluoroethylene (PTFE). Ethanol:water azeotrope (6.6 kg, which gave a permanganate time of 35 minutes and a Komarowski test result of 20 ppm of tert-butanol equivalent) and cyclohexane (0.5 kg) were fed over 15 hours onto the 40th plate of a 58-plate Oldershaw column (50 mm diameter) drying still. Organic phase from the decanter was recycled to the top of the drying still column. Aqueous phase from the decanter was fed onto the 10th plate of a 35-plate Oldershaw column (50 mm diameter) recovery still. Ethanol:cyclohexane:water azeotrope mixture was taken overhead using a reflux ratio of 7:1 and this was recycled by mixing with the feed to the drying still.

Dry ethanol final product (4.5 kg), which was collected as a liquid stream from the base of the drying still, gave a permanganate time of 22 minutes and a Komarowski test result of ca 200 ppm of tert-butanol equivalent; it also contained 0.07% w/w of water and 1 ppm of cyclohexane.

Comparing this result with that in Example 1, clearly some improvement is obtained by using PTFE flexible connections but even then the ethanol final product is downgraded by a factor of 10, which is undesirable.

Example 4

Ethanol:water azeotrope (10.9 kg, which gave a permanganate time of 35 minutes and a Komarowski test result of 20 ppm of tert-butanol equivalent) and cyclohexane (0.8 kg) were fed over 25 hours onto the 40th plate of a 58-plate Oldershaw column (50 mm diameter) drying still. Ethanol:cyclohexane:water ternary azeotrope (49.5 kg containing 4.6% w/w water) was taken overhead, condensed and the two phases which formed were separated in a decanter at 38°C.

Organic phase from the decanter was recycled to the top of the drying still column. Aqueous phase from the decanter was fed onto the 10th plate of a 35-plate Oldershaw column (50 mm diameter) recovery still. Ethanol:cyclohexane:water azeotrope mixture was taken overhead using a reflux ratio of 7:1 and this was recycled by mixing with the feed to the drying still.

The flexible connections used in both the drying and recovery stills were made of PTFE. Dry ethanol final product (7.5 kg) which was collected as a vapour stream from the base of the drying still gave a permanganate time of 24 minutes and a Komarowski test result of 18 ppm of tert-butanol equivalent; it also contained 0.04% w/w of water and 1 ppm of cyclohexane. These results demonstrate the improvement in final product quality obtainable by collecting the dry ethanol as a vapour instead of as a liquid stream from the base of the drying still.

The impurities which affected the Komarowski test concentrated up in the drying still kettle product and at the end of the experiment this stream gave a Komarowski test result of 5,000 ppm of tert-butanol equivalent.

A flow diagram of the process of the present invention is shown below.

What I claim is:

1. A process for producing substantially anhydrous ethanol of very high purity comprising azeotropically distilling aqueous ethanol together with cyclohexane as an entrainer in a drying still containing at least 50 plates, removing substantially all the water as an overhead fraction comprising a ternary azeotrope of water/cyclohexane/ethanol, and withdrawing anhydrous ethanol in the vapour phase either from a point intermediate between the level of liquid in the base of the drying still and the 5th plate, or as an overhead fraction from a further tailing still into which anhydrous liquid ethanol from the base of the drying still has been fed.

2. A process according to claim 1 wherein the drying still column contains between 60 and 70 plates.

3. A process according to any of the preceding claims wherein aqueous ethanol is fed into the upper half of the drying still column.

4. A process according to any of the preceding claims wherein the overhead ternary azeotrope product from the drying still column is condensed and the aqueous phase which separates thereby is removed by decantation and fed to a recovery still to recover the organic content of the aqueous phase.

5. A process according to claim 4 wherein the recovered organic content of the aqueous phase is recycled to the top of the drying still column.

6. A process according to any of the preceding claims wherein the temperature of the decanter is below 60$^o$C.

0001694

7. A process according to any of the preceding claims wherein dried ethanol is collected as a vapour from the base of the drying still column.

8. A process according to any of the preceding claims 1 to 6 wherein anhydrous liquid ethanol is withdrawn from the base of the drying column and fed to the lower half of a tailing still.

9. A process according to claim 8 wherein the tailing still contains between 40 and 50 plates.

10. A process according to any of the preceding claims 1 to 6, 8 and 9 wherein the tailing still is operated at a reflux ratio within the range 1 to 20:1.

ETHANOL DRYING SYSTEM

DRYING STILL

RECOVERED SOLVENT

TERNARY AZEOTROPE

CYCLOHEXANE

EtOH = H2O AZEOTROPE

H2O PHASE

H2O

RECOVERY STILL

HIGH QUALITY DRIED EtOH

BETTER QUALITY DRIED EtOH VAPOUR STREAM

POOR QUALITY DRIED EtOH LIQUID BASE PRODUCT

IMPURITIES

FURTHER RECTIFICATION STILL

# EUROPEAN SEARCH REPORT

**European Patent Office**

Application number

EP 78 300 449.2

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| – | DE - B - 1 219 011 (BASF) <br> * example * <br><br> -- | 1,3, <br> 9 | C 07 C 29/28 <br> C 07 C 31/08 |
| – | DE - B - 1 014 974 (DISTILLERS) <br> * claims 1, 2, 3, 9 * <br><br> -- | 1,4 | |
| A | Chemical Abstracts vol 46, no. 19, 1952 <br> Columbus, Ohio, USA <br> K. ZIEBORAK "Azeotropic properties of systems ethanol-water-hydrocarbons" <br> column 8947 f-g <br> & Prace Glownego Inst. Chem. Przemysl <br> no. 1; 1951 <br> page 1 to 44 <br><br> -- | | **TECHNICAL FIELDS SEARCHED (Int.Cl.²)** <br><br> C 07 C 27/30 <br> C 07 C 29/28 <br> C 07 C 31/08 |
| A | Chemical Abstracts vol. 85, no. 3, 1976 <br> Columbus, Ohio, USA <br> K.U. RIEDEL et al "Method for the purification of alcohol with cyclohexane" <br> page 501; column 1; abstract no. 18983n <br> & Branntweinwirtschaft vol. 116, <br> no. 5; 1976 <br> page 69 to 70 ---- | | **CATEGORY OF CITED DOCUMENTS** <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |

X The present search report has been drawn up for all claims

&: member of the same patent family, corresponding document

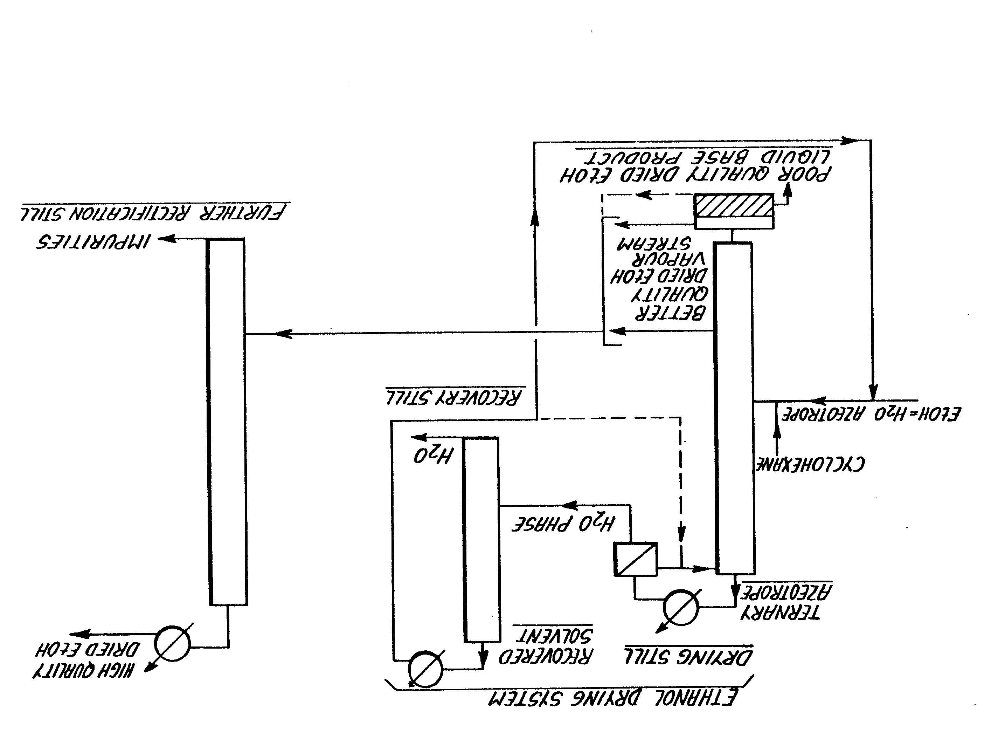

| Place of search Berlin | Date of completion of the search 14-12-1978 | Examiner KNAACK |
|---|---|---|

EPO Form 1503.1  06.78